# EUROPEAN PATENT APPLICATION

(11) **EP 4 410 958 A1**
(43) Date of publication of application: **07.08.2024**
(21) Application number: 22876522.8
(22) Date of filing: 30.09.2022
(51) Int. Cl.: C12N 5/02, C07K 7/64, C12N 5/071, C12Q 1/02

(54) **ORGANOID PRODUCTION METHOD, CULTURE MEDIUM FOR ORGANOID PRODUCTION, ORGANOID, AND TEST SUBSTANCE EVALUATION METHOD**

(30) Priority: 30.09.2021 JP 2021160758
(71) Applicant: JSR Corporation, Tokyo 105-8640 (JP)
(72) Inventor: ARAI, Kazuya, Tokyo 105-8640 (JP); ITOH, Manabu, Tokyo 105-8640 (JP); SHOJI, Kentaro, Tokyo 105-8640 (JP); SUGIMOTO, Natsumi, Tokyo 105-8640 (JP); OKADA, Ryo, Tokyo 105-8640 (JP)
(74) Representative: TBK
(86) International application number: PCT/JP2022/036633
(87) International publication number: WO 2023/054659

(57) **Abstract**

An organoid production method includes culturing a human stem cell in a culture medium containing a cyclic peptide having an amino acid sequence set forth in Formula (1) or a pharmaceutically acceptable salt of the cyclic peptide [in Formula (1), X¹ to X⁶ each represent a specific modified amino acid, X⁷ represents any amino acid residue, R is absent or represents a C-terminal modification group, n is an integer of 0 or 1, PeG is N-(2-phenylethyl)-glycine, and Nal1 is β-(1-naphthyl)-L-alanine].

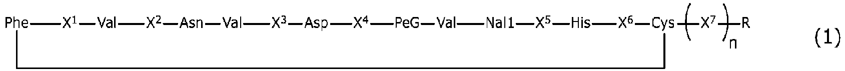

## Description

### [Technical Field]

The present invention relates to an organoid production method, a culture medium for organoid production, an organoid, and a test substance evaluation method. Priority is claimed on Japanese Patent Application No. 2021-160758, filed in Japan on September 30, 2021, the entire contents of which are incorporated herein by reference.

### [Background Art]

A TGF-β signal is known to be involved in proliferation inhibitory ability of many cells such as epithelial cells, endothelial cells, blood cells, and lymphocytes, and accumulation ability of extracellular matrix. Regulation of the TGF-β signal is necessary for producing organoids, and in particular, it is necessary to downregulate the TGF-β signal for causing cells to proliferate.

As a method of downregulating the TGF-β signal, a TGF-β receptor antagonist that binds to and inhibits a TGF-β receptor, which is one of receptors related to the TGF-β signal, has been used (refer to Patent Document 1 and Non-Patent Document 1). However, it has been reported that in a case where an antagonist such as A83-01 is used, an off-target (receptor other than TGF-β receptor) is also inhibited, and there is concern about the influence (refer to Non-Patent Document 2).

### [Citation List]

### [Patent Document]

[Patent Document 1] United States Patent Application, Publication No. 2014/0243227

### [Non-Patent Document]

[Non-Patent Document 1] Sato T., et al., Long-term expansion of epithelial organoids from human colon, adenoma, adenocarcinoma, and Barrett's epithelium, Gastroenterology, 141 (5), 1762-1772, 2011.
[Non-Patent Document 2] Vogt J., et al., The specificities of small molecule inhibitors of the TGF-beta and BMP pathways, Cell Signal., 23 (11), 1831-1842, 2011.

### [Summary of Invention]

### [Technical Problem]

The present invention is to provide an excellent organoid production method, a culture medium for the production, an organoid obtained by the production method, and a test substance evaluation method using the organoid.

### [Solution to Problem]

The present invention includes the following aspects.
[1] An organoid production method, including culturing a human stem cell in a culture medium containing a cyclic peptide having an amino acid sequence (SEQ ID NO: 1) set forth in Formula (1) or a pharmaceutically acceptable salt of the cyclic peptide (hereinafter, also referred to as "a cyclic peptide group"). 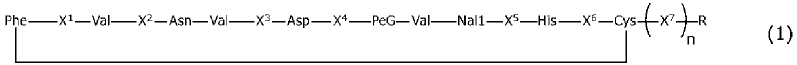 [In Formula (1), X¹ represents Tyr, W6N, W7N, or Nal1, X² represents Val, Lys, KCOp, or KCOm, X³ represents Tyr or 4Py, X⁴ represents Asp, Phe, KCOp, Glu, or KCOm, X⁵ represents Tyr or 4Py, X⁶ represents Val, Glu, KCOp, or KCOm, X⁷ represents any amino acid residue, R is absent or represents a C-terminal modification group, n is an integer of 0 or 1, PeG is N-(2-phenylethyl)-glycine, Nal1 is β-(1-naphthyl)-L-alanine, W6N is (S)-2-amino-3-(1H-pyrrolo[2,3-c]pyridin-3-yl)propanoic acid, W7N is (S)-2-amino-3-(1H-pyrrolo[2,3-b]pyridin-3-yl)propanoic acid, KCOp is N6-(4-(carboxymethyl)piperazine-1-carbonyl)-L-lysine, KCOm is N6-(methyl((2S,3R,4R,5R)-2,3,4,5,6-)pentahydroxyhexyl)carbamoyl)-L-lysine, and 4Py is 4-pyridyl-L-alanine.]
[2] The organoid production method according to [1], in which a combination of X¹ to X⁷, R, and n in Formula (1) is a combination shown in Table 1 below.

**[Table 1]**

| Peptide number | X¹ | X² | X³ | X⁴ | X⁵ | X⁶ | X⁷ | R | n |
|---|---|---|---|---|---|---|---|---|---|
| 1 | Nal1 | Val | Tyr | Asp | Tyr | Val | Gly | NH₂ | 1 |
| 2 | Nal1 | Val | Tyr | Asp | Tyr | Val | - | NH₂ | 0 |
| 3 | Tyr | Val | Tyr | Asp | Tyr | Val | Gly | NH₂ | 1 |
| 4 | W6N | Val | Tyr | Asp | Tyr | Val | Gly | NH₂ | 1 |
| 5 | W7N | Val | Tyr | Asp | Tyr | Val | Gly | NH₂ | 1 |
| 6 | Nal1 | Val | 4Py | Asp | Tyr | KCOp | Gly | NH₂ | 1 |
| 7 | Nal1 | Val | 4Py | Asp | Tyr | Val | Gly | NH₂ | 1 |
| 8 | Nal1 | Lys | 4Py | Asp | Tyr | Val | Gly | NH₂ | 1 |
| 9 | W7N | Val | 4Py | Asp | Tyr | Val | Gly | NH₂ | 1 |
| 10 | Nal1 | Val | 4Py | Asp | 4Py | Val | Gly | NH₂ | 1 |
| 11 | Nal1 | KCOp | 4Py | Asp | Tyr | Val | Gly | NH₂ | 1 |
| 12 | Nal1 | Val | 4Py | KCOp | Tyr | Val | Gly | NH₂ | 1 |
| 13 | Nal1 | Val | 4Py | Asp | Tyr | KCOp | - | NH₂ | 0 |
| 14 | Nal1 | KCOm | 4Py | Asp | Tyr | Val | Gly | NH₂ | 1 |
| 15 | Nal1 | Val | 4Py | KCOm | Tyr | Val | Gly | NH₂ | 1 |
| 16 | Nal1 | Val | 4Py | Asp | Tyr | KCOm | Gly | NH₂ | 1 |
| 17 | W7N | Val | 4Py | KCOp | Tyr | Val | Gly | NH₂ | 1 |
| 18 | W7N | Val | 4Py | KCOm | Tyr | Val | Gly | NH₂ | 1 |
| 19 | Nal1 | KCOp | 4Py | Asp | Tyr | KCOp | Gly | NH₂ | 1 |
| 20 | Nal1 | KCOm | 4Py | Asp | Tyr | KCOp | Gly | NH₂ | 1 |
| 21 | Nal1 | Val | 4Py | Phe | Tyr | KCOp | Gly | NH₂ | 1 |
| 22 | Nal1 | Val | 4Py | KCOp | Tyr | KCOp | Gly | NH₂ | 1 |
| 23 | Nal1 | Val | 4Py | KCOm | Tyr | KCOp | Gly | NH₂ | 1 |
| 24 | Nal1 | KCOm | 4Py | Phe | Tyr | KCOp | Gly | NH₂ | 1 |
| 25 | Nal1 | KCOm | 4Py | KCOp | Tyr | KCOp | Gly | NH₂ | 1 |
| 26 | Nal1 | KCOm | 4Py | KCOm | Tyr | KCOp | Gly | NH₂ | 1 |
| 27 | Nal1 | Val | 4Py | Asp | Tyr | Glu | Gly | NH₂ | 1 |
| 28 | Nal1 | KCOm | 4Py | Asp | Tyr | Glu | Gly | NH₂ | 1 |
| 29 | Nal1 | KCOm | 4Py | Glu | Tyr | Glu | Gly | NH₂ | 1 |
| 30 | Nal1 | KCOm | 4Py | KCOm | Tyr | Glu | Gly | NH₂ | 1 |
| 31 | Nal1 | Val | 4Py | Asp | Tyr | KCOp | Gly-Lys | PEG-NH₂ | 1 |

| | | | | | | | | | |
|---|---|---|---|---|---|---|---|---|---|
| In Table 1, "PEG" represents polyethylene glycol chain. | | | | | | | | | |

[3] The organoid production method according to [1] or [2], in which in the amino acid sequence set forth in Formula (1), X¹ is Nal1, X² is Val, X⁴ is Asp, and X⁵ is Tyr.
[4] The organoid production method according to any one of [1] to [3], in which a bond between Phe and Cys in the amino acid sequence represented by Formula (1) is a bond via a thioether bond.
[5] The organoid production method according to any one of [1] to [4], in which X⁷ of the amino acid sequence represented by Formula (1) includes Gly.
[6] The organoid production method according to any one of [1] to [5], in which the total concentration of the cyclic peptide and the pharmaceutically acceptable salt of the cyclic peptide contained in the culture medium is 0.1 nM to 10 µM.
[7] The organoid production method according to any one of [1] to [6], in which the culture medium further contains a Wnt signal activator.
[8] The organoid production method according to any one of [1] to [7], in which the culture medium further contains an epidermal growth factor (EGF), a fibroblast growth factor (FGF), an insulin-like growth factor (IGF), a hepatocyte growth factor (HGF), or a combination thereof.
[9] The organoid production method according to any one of [1] to [8], in which the culture medium further contains a bone morphogenetic protein (BMP) inhibitor.
[10] The organoid production method according to any one of [1] to [9], in which the culture medium further contains a Rho-kinase (ROCK) signaling pathway inhibitor.
[11] The organoid production method according to any one of [1] to [10], in which the culture medium does not substantially contain an antagonist of ALK5, ALK4, ALK7, or a combination thereof.
[12] The organoid production method according to any one of [1] to [11], in which the human stem cell is cultured while being brought into contact with an extracellular matrix.
[13] A culture medium for organoid production, containing a cyclic peptide having an amino acid sequence (SEQ ID NO: 1) set forth in Formula (1) or a pharmaceutically acceptable salt of the cyclic peptide. 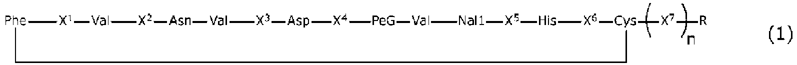 [In Formula (1), X¹ represents Tyr, W6N, W7N, or Nal1, X² represents Val, Lys, KCOp, or KCOm, X³ represents Tyr or 4Py, X⁴ represents Asp, Phe, KCOp, Glu, or KCOm, X⁵ represents Tyr or 4Py, X⁶ represents Val, Glu, KCOp, or KCOm, X⁷ represents any amino acid residue, R is absent or represents a C-terminal modification group, n is an integer of 0 or 1, PeG is N-(2-phenylethyl)-glycine, Nal1 is β-(1-naphthyl)-L-alanine, W6N is (S)-2-amino-3-(1H-pyrrolo[2,3-c]pyridin-3-yl)propanoic acid, W7N is (S)-2-amino-3-(1H-pyrrolo[2,3-b]pyridin-3-yl)propanoic acid, KCOp is N6-(4-(carboxymethyl)piperazine-1-carbonyl)-L-lysine, KCOm is N6-(methyl((2S,3R,4R,5R)-2,3,4,5,6-)pentahydroxyhexyl)carbamoyl)-L-lysine, and 4Py is 4-pyridyl-L-alanine.]
[14] An organoid obtained by the organoid production method according to any one of [1] to [12].
[15] A test substance evaluation method including: a step of bringing the organoid according to [14] into contact with a test substance; and a step of evaluating an influence exerted by the test substance on the organoid.

### [Advantageous Effects of Invention]

According to the present invention, it is possible to provide an organoid production method having excellent proliferative properties, a culture medium used in the production method, an organoid obtained by the production method, and a test substance evaluation method using the organoid.

### [Brief Description of Drawings]

FIG. 1 is a microscopic image showing a result of Experimental Example 2.
FIG. 2 is a graph showing a result of Experimental Example 3.
FIG. 3 is a graph showing a result of Experimental Example 3.
FIG. 4 is a graph showing a result of Experimental Example 4.
FIG. 5 is a microscopic image showing a result of Experimental Example 5.
FIG. 6 is a microscopic image showing a result of Experimental Example 5.
FIG. 7 is a microscopic image showing a result of Experimental Example 6.

### [Description of Embodiments]

Hereinafter, the present invention will be described in more detail with reference to embodiments, but the present invention is not limited to the following embodiments.

Unless stated otherwise, each component exemplified in the present specification, for example, a component included in a culture medium can be used alone, or two or more kinds thereof may be used in combination.

A, B, a, and b indicate any numerical values, and in a case of a relationship of A > B > a > b, in the present specification, a notation representing a numerical value range such as the phrase "A to B" is the same as the phrase "equal to or more than A, equal to or less than B". In addition, in the present specification, a notation representing a numerical value range such as the phrase "A to B, preferably a to b" is the same as the phrases "equal to or more than A, equal to or less than B", "equal to or more than A, equal to or less than b", "equal to or more than a, equal to or less than B", and "equal to or more than a, equal to or less than b".

In the present specification, the phrases "culture medium containing substance X" and "in the presence of substance X" mean a culture medium to which an exogenous substance X is added, a culture medium containing an exogenous substance X, or in the presence of an exogenous substance X. That is, in a case where a cell or tissue present in the culture medium expresses, secretes, or produces the substance X endogenously, an endogenous substance X is distinguished from the exogenous substance X, and a culture medium not containing an exogenous substance X does not fall under the category of "culture medium containing a substance X" even if the culture medium contains an endogenous substance X.

### Organoid production method

In one embodiment, the present invention provides an organoid production method including culturing a human stem cell in a culture medium containing a cyclic peptide having an amino acid sequence set forth in Formula (1) or a pharmaceutically acceptable salt thereof.

In a case where a human stem cell is cultured in the culture medium, the human stem cell proliferates and forms an organoid containing both the stem cell and a differentiated cell.

In the present specification, the amino acid includes a natural amino acid and an unnatural amino acid. Hereinafter, a "cyclic peptide having an amino acid sequence (SEQ ID NO: 1) set forth in Formula (1) or a pharmaceutically acceptable salt thereof' may be referred to as a "cyclic peptide group".

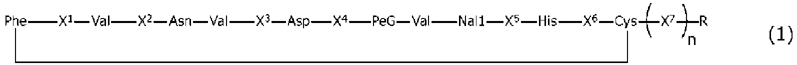

In Formula (1), X¹ represents Tyr, W6N, W7N, or Nall, X² represents Val, Lys, KCOp, or KCOm, X³ represents Tyr or 4Py, X⁴ represents Asp, Phe, KCOp, Glu, or KCOm, X⁵ represents Tyr or 4Py, X⁶ represents Val, Glu, KCOp, or KCOm, X⁷ represents any amino acid residue, R is absent or represents a C-terminal modification group, n represents an integer of 0 or 1, PeG is N-(2-phenylethyl)-glycine, Nal1 is β-(1-naphthyl)-L-alanine, W6N is (S)-2-amino-3-(1H-pyrrolo[2,3-c]pyridin-3-yl)propanoic acid, W7N is (S)-2-amino-3-(1H-pyrrolo[2,3-b]pyridin-3-yl)propanoic acid, KCOp is N6-(4-(carboxymethyl)piperazine-1-carbonyl)-L-lysine, KCOm is N6-(methyl((2S,3R,4R,5R)-2,3,4,5,6-)pentahydroxyhexyl)carbamoyl)-L-lysine, and 4Py is 4-pyridyl-L-alanine.

As will be described later in Examples, according to the organoid production method of the present embodiment, a human stem cell can be cultured without using a TGF-β receptor antagonist such as A83-01. Many antagonists are known to act on a plurality of receptors, and for example, in the case of A83-01, it is known to strongly act on not only ALK5 which is a TGF-β receptor, but also VEGFR, RIPK2, MINK1, p38α MAPK, PKD1, and FGF-R1. On the other hand, since the cyclic peptide group acts on a ligand of the TGF-β receptor, it is possible to eliminate an off-target influence of the TGF-β receptor antagonist. Here, the ligand of the TGF-β receptor refers to a ligand having an agonist action. In addition, since the cyclic peptide group is estimated to have substantially no action on the off-target, the cyclic peptide group in the culture medium can culture a human stem cell at a concentration lower than the required concentration of the TGF-β receptor antagonist.

The cyclic peptide group is estimated to be capable of binding to, preferably specifically binding to a ligand of an exogenous or endogenous TGF-β receptor.

The organoid production method of the present embodiment can culture a human stem cell at a concentration lower than the required concentration of a TGF-β receptor antagonist by using a cyclic peptide group instead of the TGF-β receptor antagonist in cell culture which has been carried out using a TGF-β receptor antagonist such as A83-01 in the related art.

In the organoid production method of the present embodiment, the human stem cell means a human cell or a cell derived from a human, which has self-replication ability and differentiation ability. Examples of the human stem cell include a human somatic stem cell such as a human epithelial stem cell, a human mesenchymal stem cell, a human vascular cell, or a human cancer cell; a human pluripotent stem cell such as a human induced pluripotent stem cell (hiPSC) and a human embryonic stem cell (hESC); and a human progenitor cell which is in a stage of being differentiated from a human stem cell into a specific somatic cell or reproductive cell and has a differentiation ability into a specific tissue or organ. In a culture method of the present embodiment, a cell population containing human stem cells can be used as the human stem cell.

In the organoid production method of the present embodiment, in a case where a human stem cell forms a cell aggregate, a dispersion treatment is generally carried out. The dispersion refers to separating cells into a cell population of 100 or less, preferably a cell population of 50 or less, and more preferably a single cell by a dispersion treatment such as an enzyme treatment or a physical treatment. Examples of the dispersion treatment include a mechanical dispersion treatment; a treatment using a cell dispersion liquid such as trypsin, collagenase, papain, and ethylenediamine tetraacetic acid. Before the dispersion treatment is carried out, a treatment with a cell protecting agent such as heparin, a ROCK inhibitor, and an insulin-like growth factor can be carried out to prevent cell death.

A cyclic peptide is characterized to have Asn-Val-Tyr-Asp (SEQ ID NO: 2) or Asn-Val-4Py-Asp in seq. 5 to 8 and Val-Nal1-Tyr-His or Val-Nal1-4Py-His in seq. 11 to 14 in Formula (1-1) (SEQ ID NO: 1). Peptides having these amino acid sequences have activity in ligands of TGF-β receptors.

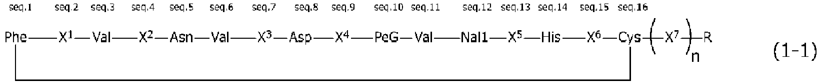

seq. 2, 4, 9, and 15 are each constituted of residues selected from the group consisting of a hydrophilic amino acid, a hydrophobic amino acid, and an aromatic amino acid, and by appropriately adjusting a combination of these residues, it is possible to control solubility in a culture medium while maintaining a higher-order structure of the cyclic peptide.

In Formula (1) or Formula (1-1), X⁷ is a linker at a C-terminal of the peptide, and can be one or a plurality of any amino acid residues. X⁷ is used for binding to puromycin, a low molecular weight compound, another peptide, a protein, and the like in a translation synthesis system. Examples of X⁷ include those containing Gly and Gly-Lys. In addition, n is 0 or 1.

In addition, R is absent or represents a modification group of a C-terminal carboxyl group, and is preferably a modification group that forms a structure of -COOR with a C-terminal carboxyl group, removes a negative charge of the carboxyl group, and adds a positive charge. Examples of such a modification group include an amino group, an amide group, an aminoethyl group, a pyrazolidine group, a piperidine group, an imidazolidine group, and a piperazine group. In addition, R can contain a polyethylene glycol chain.

As an optimal amino acid sequence of the cyclic peptide, a combination of X¹ to X⁶ of the cyclic peptide is preferably any one of the combinations shown in Table 1 above, and is more preferably a combination in which X¹ is Nal1, X² is Val, X⁴ is Asp, X⁵ is Tyr, X⁶ is Val or KCOp.

In the present specification, the cyclic peptide can be produced by a known production method such as a chemical synthesis method such as a liquid phase method, a solid phase method, a hybrid method combining a liquid phase method and a solid phase method; and a genetic recombination method.

In the solid phase method, for example, a cyclic peptide is synthesized by the following method. First, a hydroxyl group of a resin having a hydroxyl group and a carboxy group of a first amino acid in which an α-amino group is protected by a protective group (generally, the C-terminal amino acid of the target peptide) are subjected to an esterification reaction. As an esterification catalyst, it is possible to use a known dewater condensation agent such as 1-mesitylenesulfonyl-3-nitro-1,2,4-triazole (MSNT), dicyclohexylcarbodiimide (DCC), and diisopropylcarbodiimide (DIPCDI).

Next, the protective group of the α-amino group of the first amino acid is eliminated, and a second amino acid in which all functional groups other than the carboxy group of the main chain are protected is added, and the carboxy group is activated to bind the first and second amino acids. In addition, the α-amino group of the second amino acid is deprotected, a third amino acid in which all functional groups other than the carboxy group of the main chain are protected is added, and the carboxy group is activated to bind the second amino acid and a third amino acid. This is repeated, and in a case where a peptide having a targeted length is synthesized, all functional groups are deprotected.

Examples of the resin that is used in the solid phase method include Merrifield resin, MBHA resin, Cl-Trt resin, SASRIN resin, Wang resin, Rink amide resin, HMFS resin, Amino-PEGA resin (Merck Group), HMPA-PEGA resin (Merck Group), and the like. These resins can be used after being washed with a solvent (dimethylformamide (DMF), 2-propanol, methylene chloride, and the like).

Examples of the protective group for the α-amino group include a benzyloxycarbonyl (Cbz or Z) group, a tert-butoxycarbonyl (Boc) group, a fluorenylmethoxycarbonyl (Fmoc) group, a benzyl group, an allyl group, an allyloxycarbonyl (Alloc) group, and the like. The Cbz group can be deprotected by hydrofluoric acid, hydrogenation, and the like, the Boc group can be deprotected by trifluoroacetic acid (TFA), and the Fmoc group can be deprotected by treatment with piperidine.

For protection of the α-carboxy group, methyl ester, ethyl ester, benzyl ester, tert-butyl ester, cyclohexyl ester, and the like can be used.

As other functional groups of amino acids, a hydroxy group of serine or threonine can be protected with a benzyl group or a tert-butyl group, and a hydroxy group of tyrosine can be protected with a 2-bromobenzyloxycarbonyl group or a tert-butyl group. An amino group of a lysine side chain and a carboxy group of glutamic acid or aspartic acid can be protected in the same manner as the α-amino group and the α-carboxy group.

Activation of the carboxy group can be carried out using a condensation agent. Examples of the condensation agent include dicyclohexylcarbodiimide (DCC), diisopropylcarbodiimide (DIPCDI), 1-ethyl-3-(3-dimethylaminopropyl)carbodiimide (EDC or WSC), (1H-benzotriazol-1-yloxy)tris (dimethylamino)phosphonium hexafluorophosphate (BOP), 1-[bis(dimethylamino)methyl]-1H-benzotriazolium-3-oxidehexafluorophosphate (HBTU), and the like.

Severing of the peptide chain from the resin can be carried out by a treatment with an acid such as TFA, hydrogen fluoride (HF), and the like.

Production of a cyclic peptide by a genetic recombination method (a translation synthesis system) can be carried out using a nucleic acid encoding a targeted cyclic peptide. The nucleic acid encoding a cyclic peptide can be DNA or RNA.

The nucleic acid encoding a cyclic peptide can be prepared by a known method. For example, the nucleic acid can be synthesized with an automatic synthesizer. A restriction enzyme recognition site may be added in order to insert the obtained DNA into a vector, or a base sequence encoding an amino acid sequence for cleaving the synthesized peptide chain with an enzyme and the like may be incorporated.

In order to suppress degradation by host-derived protease, a chimeric protein expression method can be used in which the targeted peptide is expressed as a chimeric peptide together with another peptide. In this case, as the nucleic acid, a nucleic acid encoding a targeted peptide and a peptide binding thereto is used.

Subsequently, an expression vector containing a nucleic acid encoding a peptide is prepared. The nucleic acid encoding a peptide can be inserted downstream of a promoter of the expression vector as it is or after digestion with a restriction enzyme or addition of a linker or the like. Examples of the vector include E. coli-derived plasmid (pBR322, pBR325, pUC12, pUC13, pUC18, pUC19, pUC118, pBluescript II, and the like), Bacillus subtilis-derived plasmid (pUB 110, pTP5, pC1912, pTP4, pE194, pC194, and the like), yeast-derived plasmid (pSH19, pSH15, YEp, YRp, YIp, YAC, and the like), bacteriophage (e-phage, M13 phage, and the like), virus (retrovirus, vaccinia virus, adenovirus, adeno-associated virus (AAV), cauliflower mosaic virus, tobacco mosaic virus, baculovirus, and the like), cosmid, and the like.

The promoter can be appropriately selected depending on the kind of a host. In a case where a host is an animal cell, for example, an SV40 (simian virus 40)-derived promoter and a CMV (cytomegalovirus)-derived promoter can be used. In a case where the host is E. coli, a trp promoter, a T7 promoter, a lac promoter, and the like can be used.

In the expression vector, nucleic acids encoding DNA replication initiation points (ori), selection markers (antibiotic resistance, auxotrophy, and the like), enhancers, splicing signals, poly A addition signals, and tags (FLAG, HA, GST, GFP, and the like) can be incorporated.

Subsequently, an appropriate host cell is transformed with the expression vector. A host can be appropriately selected depending on the relationship with the vector, and for example, E. coli, Bacillus subtilis, bacteria of the genus Bacillus, yeast, insect living organism, insect cell, animal cell, and the like are used. As the animal cell, for example, HEK293T cells, CHO cells, COS cells, myeloma cells, HeLa cells, and Vero cells can be used. Transformation can be carried out by a known method such as a lipofection method, a calcium phosphate method, an electroporation method, a microinjection method, a particle gun method, and the like depending on the kind of the host. By culturing a transformant according to a conventional method, a targeted peptide is expressed.

In the purification of peptides from the culture of the transformant, first, cultured cells are recovered, suspended in an appropriate buffer, and then disrupted by a method such as ultrasonic treatment, freeze-thaw, or the like, and subjected to centrifugation or filtration to obtain a crude extraction liquid. In a case where peptides are secreted into the culture solution, a supernatant is recovered.

The purification of peptides from the crude extraction liquid or the culture supernatant may be carried out by a known method or a method similar thereto (for example, salting out, dialysis method, ultrafiltration method, gel filtration method, SDS-PAGE method, ion exchange chromatography, affinity chromatography, reverse phase high performance liquid chromatography, and the like).

The obtained peptide can be converted from a free form into a salt or from a salt into a free form by a known method or a method similar thereto.

Examples of the translation synthesis system include a cell-free translation system. Examples of the cell-free translation system include a ribosomal protein, an aminoacyl tRNA synthetase (ARS), a ribosomal RNA, an amino acid, an rRNA, a GTP, an ATP, a translation initiation factor (IF), an elongation factor (EF), a termination factor (RF), a ribosome regeneration factor (RRF), and other factors necessary for translation. An E. coli extraction liquid or a wheat germ extraction liquid may be added to increase an expression efficiency. In addition, a rabbit erythrocyte extraction liquid or an insect cell extraction liquid can be added. In the cell-free translation system, for example, an mRNA display method can be carried out, and in this case, puromycin that is responsible for the binding between a template mRNA and a translated peptide can be connected via a linker.

By continuously supplying energy to a system including these cells using dialysis, it is possible to produce peptides from several hundred µg to several mg/mL. It is possible to use a system including RNA polymerase to also carry out transcription from gene DNA. As a commercially available cell-free translation system, it is possible to use RTS-100 (registered trademark) of Roche Diagnostics K.K., PURESYSTEM (registered trademark) of PGI Co., Ltd., and the like as a system derived from E. coli, and those of ZOEGENE Corporation or CellFree Sciences Co., Ltd. as a system using a wheat germ extraction liquid. According to the cell-free translation system, an expression product can be obtained in a highly pure form without purification.

In the cell-free translation system, instead of aminoacyl tRNA synthesized by natural aminoacyl tRNA synthetase, it is possible to use artificial aminoacyl tRNA in which unnatural amino acid or hydroxy acid is linked (acylated) to tRNA. Such aminoacyl tRNAs can be synthesized using artificial ribozymes such as flexizyme.

By using tRNA linked with an unnatural amino acid or a hydroxy acid, a desired codon can be translated in association with the unnatural amino acid or the hydroxy acid.

The unnatural amino acids in the cyclic peptide are shown in Table 2 below. In Table 2, the three-dimensional structure of the chemical formula is omitted.

**[Table 2]**

| Name | Amino acid | CAS No. | Chemical formula |
|---|---|---|---|
| PeG | N-(2-phenylethyl)-glycine | 7738-38-7 | 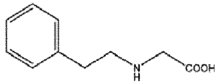 |
| Nal1 | β-(1-naphthyl)-L-alanine | 55516-54-6 | 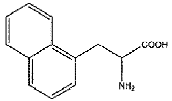 |
| W6N | (S)-2-amino-3-(1H-pyrrolo[ 2,3-c])yridine-3-yl)propano ic acid | 149704-63-2 | 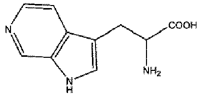 |
| W7N | (S)-2-amino-3-(1H-pyrrolo[ 2,3-b]pyridine-3-yl)propano ic acid | 49758-35-2 | 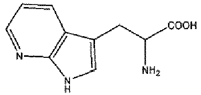 |
| KCOp | N6-(4-(carboxymethyl)piper azine-1-carbonyl)-L-lysine | - | 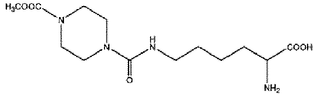 |
| KCOm | N6-(methyl((2S,3R,4R,5R)-2,3,4,5,6-)pentahydroxyhex yl)carbamoyl)-L-lysine | - | 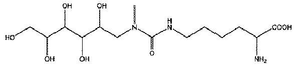 |
| 4Py | 4-pyridyl-L-alanine | 1956-21-4 | 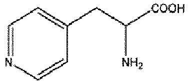 |

The cyclization of peptides is carried out by forming a thioether bond by a cyclization reaction between a thiol group of Cys of seq. 16 and, for example, a chloroacetyl group (Cl-CH₂-CO-NH-C(Bzl)-COO) of chloroacetylated Phe of seq. 1 or Cl-R-CO-NH-C(Bzl)-COO- (here, R is an alkanediyl group). In this case, the bond between Phe and Cys in the amino acid sequence represented by Formula (1) is a bond through a thioether bond. For cyclization, a complex between chloroacetylated Phe and tRNA can be generated by flexizyme and used in a translation synthesis system.

Since the thioether bond formed between the chloroacetyl group and thiol is less likely to be degraded even under reducing conditions, the half-life is long.

The cyclic peptide has a binding ability to a ligand of a TGF-β receptor. The TGF-β receptor can be ALK5, ALK4, ALK7, or a combination thereof. NCBI accession numbers of human ALK5 are NP_001124388.1, NP_001293139.1, NP_004603.1, and the like. In addition, NCBI accession numbers of human ALK4 are NP_004293.1, NP_064732.3, NP_064733.3, and the like. In addition, NCBI accession numbers of human ALK7 are NP_001104501.1, NP_001104502.1, NP_001104503.1, NP_660302.2, and the like.

Examples of the ligand of the TGF-β receptor to which the cyclic peptide binds include TGF-β1, TGF-β2, and TGF-β3, and TGF-β1 is preferable. The NCBI accession number of human TGF-β1 is NP_000651.3 and the like. The NCBI accession numbers of human TGF-β2 are NP_001129071.1, NP_003229.1, and the like. The NCBI accession numbers of human TGF-β3 are NP_001316867.1, NP_001316868.1, NP_003230.1, and the like.

The cyclic peptide may be a derivative. Examples of a derivative of the cyclic peptide include those in which a polyethylene glycol chain or the like has been added to any one of the amino acids constituting the cyclic peptide; those in which any amino acid constituting the cyclic peptide is converted into a D form; and a complex in which a low molecular weight compound, another peptide, a protein, or the like is bound via a linker.

Examples of the pharmaceutically acceptable salt of a cyclic peptide include a sodium salt, a potassium salt, a calcium salt, a magnesium salt, an ammonium salt, a salt with trimethylamine, a salt with pyridine, a salt with ethanolamine, a salt with tartaric acid, a salt with citric acid, a salt with succinic acid, and a salt with malic acid. The pharmaceutically acceptable salt of the cyclic peptide can be produced by subjecting the cyclic peptide to salt exchange. The pharmaceutically acceptable salt includes a biologically acceptable salt.

In the organoid production method of the present embodiment, the concentration of the cyclic peptide contained in the culture medium (the total concentration of the cyclic peptide, a derivative thereof, and the pharmaceutically acceptable salt thereof) is generally 0.1 nM to 10 µM, for example, 0.1 nM to 1,000 µM, and is appropriately adjusted depending on the cell type.

In the organoid production method of the present embodiment, the culture medium is generally prepared by adding a specific cyclic peptide to a basal medium. In addition, it is possible to appropriately add various components shown below to the culture medium. Examples of the basal medium include Dulbecco's modified Eagle medium (DMEM), minimum essential medium (MEM), Knockout-DMEM (KO-DMEM), Glasgow minimum essential medium (G-MEM), basal medium Eagle (BME), DMEM/F12, Advanced DMEM/F12, Iscove's modified Dulbecco's medium, Ham's F-10, Ham's F-12, 199 medium, RPMI1640 medium, and the like. In the present specification, the basal medium refers to a culture medium containing carbon sources such as amino acids, antioxidants, minerals, and glucose, and optionally containing components essential for at least culturing, such as serum, fatty acids, proteins such as insulin or transferrin, and the like.

The culture medium can contain a Wnt signal activator. Examples of the Wnt signal activator include a Wnt family member, a GSK inhibitor or Lgr5 agonist, or a combination thereof.

Examples of the Wnt family members include Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, Wnt16, and the like. Examples of the Wnt family member also include a complex with afamin, which is a stabilizer thereof.

Examples of the GSK inhibitor include GSK-3β inhibitor, for example, CHIR99021 (CAS number: 252917-06-9), Kenpaullone (CAS number: 142273-20-9), and 6-Bromoindirubin-3'-oxime (BIO, CAS number: 667463-62-9).

Examples of the Lgr5 agonist include R-spondin family. Examples of the R-spondin family include R-spondin1, R-spondin2, R-spondin3, R-spondin4, and the like, and among these, R-spondin1 is preferable.

In a case where CHIR99021 is used as the Wnt signal activator, the concentration of CHIR99021 contained in the culture medium is generally 0.1 µM to 30 µM and preferably 0.2 µM to 20 µM.

In a case where Wnt3a is used as the Wnt signal activator, the concentration of Wnt3a contained in the culture medium is generally 0.1 ng/mL to 20 ng/mL and preferably 0.2 ng/mL to 10 ng/mL.

The culture medium can contain epidermal growth factor (EGF), fibroblast growth factor (FGF), insulin-like growth factor (IGF), hepatocyte growth factor (HGF), or a combination thereof.

The concentration of EGF contained in the culture medium is generally 5 ng/mL to 500 ng/mL, preferably 25 ng/mL to 300 ng/mL, and more preferably 50 ng/mL to 100 ng/mL.

Examples of FGF include FGF2, FGF4, FGF7, and FGF10, and among these, FGF10 is preferable. The concentration of FGF contained in the culture medium is generally 20 ng/mL to 500 ng/mL, preferably 50 ng/mL to 500 ng/mL, and more preferably 100 ng/mL to 500 ng/mL.

As the IGF, IGF1 is preferable. The concentration of IGF contained in the culture medium is generally 5 ng/mL to 1,000 ng/mL, preferably 10 ng/mL to 1,000 ng/mL, and more preferably 50 ng/mL to 1,000 ng/mL.

The concentration of HGF contained in the culture medium is generally 1 ng/mL to 100 ng/mL, preferably 10 ng/mL to 100 ng/mL, more preferably 20 ng/mL to 100 ng/mL, and furthermore preferably 40 ng/mL to 100 ng/mL.

The culture medium can contain a bone morphogenetic protein (BMP) inhibitor. The BMP inhibitor is preferably Noggin. The concentration of the BMP inhibitor contained in the culture medium is generally 10 to 100 ng/mL, preferably 20 to 100 ng/mL, and more preferably 50 to 100 ng/mL.

The culture medium can contain a ROCK signaling pathway inhibitor. Examples of the ROCK signaling pathway inhibitor include Y-27632 (CAS number: 146986-50-7), Fasudil (CAS number: 105628-07-7), Y39983 (CAS number: 203911-26-6), Wf-536 (CAS number: 539857-64-2), SLx-2119 (CAS number: 911417-87-3), Azabenzimidazole-aminofurazans (CAS number: 850664-21-0), DE-104, H-1152P (CAS number: 872543-07-6), Blebbistatin (CAS number: 856925-71-8), and the like.

The concentration of the ROCK signaling pathway inhibitor contained in the culture medium is generally 1 µM to 20 µM, and preferably 5 µM to 15 µM.

In addition to the above-described components, the culture medium can contain, for example, gastrin (or an appropriate substitute such as Leu15-gastrin I); a p38 inhibitor such as SB202190 (CAS number: 152121-30-7) and Doramapimod (CAS number: 285983-48-4); an antibacterial agent such as a penicillin-based antibiotic, a cephem-based antibiotic, a macrolide-based antibiotic, and a tetracycline-based antibiotic; IL-6 family cytokines such as interleukin-6 (IL-6), interleukin-11 (IL-11), Oncostatin M (OSM), leukemia inhibitory factor (LIF), Cardiotrophin-1 (CT-1), and ciliary neurotrophic factor (CNTF); retinoic acid; nicotinamide; tumor cell growth factor-α (TGF-α) such as forscholine; γ-secretase inhibitor (DAPT); dimethylsulfoxide (DMSO); dexamethasone (Dex); fatty acids such as lauric acid, myristic acid, linoleic acid, linolenic acid, and oleic acid; and a combination thereof.

The culture medium can contain glutamic acid-containing supplements such as B27 supplement and GlutaMax series, which are sold by Thermo Fisher Scientific, Inc. and the like, an amino acid aqueous solution such as MEM Non-Essential Amino Acids Solution, and a supplement such as N2 supplement.

In the organoid production method of the present embodiment, it is preferable that the culture medium does not substantially contain an antagonist of the TGF-β receptor of any of ALK5, ALK4, ALK7, or a combination thereof. Here, the phrase "does not substantially contain" means not intentional mixing as an effective component, or allowing unintentional mixture to the extent that the effect is negligible. Specifically, the concentration of the TGF-β receptor antagonist contained in the culture medium is 1 nM or less, preferably 0.1 nM or less, and more preferably 0.01 nM or less.

Examples of the TGF-β receptor antagonist include A83-01 (CAS number: 909910-43-6), SB-431542 (CAS number: 301836-41-9), SB-505124 (CAS number: 694433-59-5), SB-525334 (CAS number: 356559-20-1), LY364947 (CAS number: 396129-53-6), SD208 (CAS number: 627536-09-8), SJN2511 (CAS number: 446859-33-2), and the like. Examples of the TGF-β receptor ligand include TGF-β1, TGF-β2, TGF-β3, and the like.

In the organoid production method of the present embodiment, a human stem cell is preferably cultured while being brought into contact with an extracellular matrix.

Examples of a method of culturing a human stem cell while being brought into contact with an extracellular matrix include a method of embedding a human stem cell in an extracellular matrix (ECM) and culturing thereof, a method of culturing a human stem cell by mixing ECM with a culture medium, a method of culturing a human stem cell by coating ECM on a culture surface of a culture container, and the like. Examples of the ECM include a component contained in a basal membrane and a glycoprotein present in an intercellular space. Examples of the component contained in the basal membrane include type IV collagen, laminin, heparan sulfate proteoglycan, entactin, and the like. Examples of the glycoprotein present in the intercellular space include collagen, laminin, entactin, fibronectin, and heparin sulfate. As the ECM, a commercially available product containing ECM can be used. Examples of a commercially available product including ECM include Matrigel (registered trademark, Corning Inc.), human laminin (Sigma-Aldrich Corporation), and the like.

The culture medium mixed with ECM can be prepared by carrying out mixing in an amount in which a volume of ECM is generally 1% by volume or more, preferably 5% by volume or more and 100% by volume or less, and more preferably 10% by volume or more and 90% by volume or less with respect to a volume of components other than ECM in the culture medium. Examples of the method of mixing ECM include a pipetting method on an ice bath. The mixing means a state in which ECM is not visually observed in the culture medium.

The culture medium can be exchanged about once every 1 to 5 days. The culture is generally carried out under a temperature condition of 30°C to 50°C, preferably 32°C to 48°C, and more preferably 34°C to 46°C. The culture is performed in an atmosphere in which a content proportion of the carbon dioxide is generally 1% by volume to 15% by volume, preferably 2% by volume to 14% by volume, and more preferably 3% by volume to 13% by volume or less.

### Culture medium for organoid production

In one embodiment, the present invention provides a culture medium for organoid production containing a cyclic peptide having an amino acid sequence set forth in Formula (1), or a pharmaceutically acceptable salt thereof.

The culture medium for organoid production of the present embodiment can also be referred to as a culture medium for organoid culturing. The culture medium of the present embodiment makes it possible to favorably culture a human stem cell and produce an organoid.

The culture medium is prepared by adding various components to the basal medium. The basal medium and various components are the same as those described above.

### Organoid

In one embodiment, the present invention provides an organoid obtained by the above-described organoid production method. In the present specification, the organoid means a self-organizing three-dimensional cell culture formed in vitro and obtained by culturing a stem cell. The cell culture can also have a structure close to an organ. The organoid of the present embodiment can be cultured in the absence of a TGF-β receptor antagonist. Therefore, it is considered that, unlike the organoid obtained using a TGF-β receptor antagonist in the related art, the organoid of the present embodiment is not affected by off-target inhibition by a TGF-β receptor antagonist and more reflects a state of a cell in vivo.

Examples of the organoid of the present embodiment include a colon organoid, a small intestinal organoid, a liver organoid, a bile duct organoid, a brain organoid, an ovarian cancer organoid, a retinal organoid, and the like.

The organoid of the present embodiment is considered to have a difference in the gene expression profile and the like as compared with an organoid obtained by a production method other than the organoid production method of the present embodiment. However, it is extremely difficult to specify such a difference, and specify whether the organoid is an organoid obtained by a production method other than the organoid production method of the present embodiment or the organoid of the present embodiment by the difference of the gene expression profile and the like, and thus such specifications are impractical. Therefore, it is realistic to carry out specification by the production method.

### Test substance evaluation method

In one embodiment, the present invention provides the test substance evaluation method including a step of bringing a test substance into contact with the above-described organoid (hereinafter, also referred to as "step 1"), and a step of evaluating an influence exerted by the test substance on the organoid (hereinafter, also referred to as "step 2").

Examples of the test substance include a natural compound library, a synthetic compound library, an existing drug library, and a metabolite library. In addition, a new drug can be used as the test substance.

In step 1, the organoid can be used to construct a cell-based assay system such as a transwell type assay system, or a biomimetic system such as an organ-on-a-chip, such that the organoid can be brought into contact with the test substance.

In step 2, the influence exerted by the test substance on the organoid can evaluated by Western blotting, ELISA, immunostaining, and the like.

### [Examples]

Hereinafter, the present invention will be described in more detail with reference to experimental examples, but the present invention is not limited to the following experimental examples. In the following experimental examples, devices that had been subjected to sterilization treatment were used in all of the experiments.

### [Experimental Example 1]

### (Preparation of culture medium for culturing colorectal epithelial stem cell)

To a basal medium, each component was added to have the concentration shown in Table 3 below to prepare a culture medium. In Table 3, "CM" means conditioned medium. Advanced DMEM/F12 was used as a basal medium. As the peptide, peptide names #7401 and #2392 were used (hereinafter, each may be referred to as "#7401 peptide" and "#2392 peptide"). Regarding the #7401 peptide, a chemical formula is shown in Formula (2-1), and an amino acid sequence (SEQ ID NO: 3) is shown in Formula (2-2). In addition, regarding the #2392 peptide, a chemical formula is shown in Formula (3-1), and an amino acid sequence (SEQ ID NO: 4) is shown in Formula (3-2).

**[Table 3]**

| Component | Concentration |
|---|---|
| HEPES | 10 nM |
| GlutaMAX | 2 nM |
| Gibco^{™} Penicillin-Streptomycin (5,000 U/mL) | Penicillin 100 U/mL |
| (Thermo Fisher Scientific, Inc.) | Streptomycin 100 mg/mL |
| [Leucine15]-gastrin I | 10 nM |
| N-acetyl-L-cysteine | 1 mM |
| EGF | 50 ng/mL |
| IGF1 | 100 ng/mL |
| FGF2 | 50 ng/mL |
| Wnt3a (Wnt3a CM) | 20 v/v% (to basal medium) |
| R-spondin1 (R-spondin1 CM) | 4 v/v% (to basal medium) |
| Noggin (Noggin CM) | 2 v/v% (to basal medium) |
| Gibco^{™} B27 supplement (Thermo Fisher Scientific, Inc.) | Appropriate amount |
| A83-01 or peptide | 0.5 nM, 5 nM, or 50 nM |

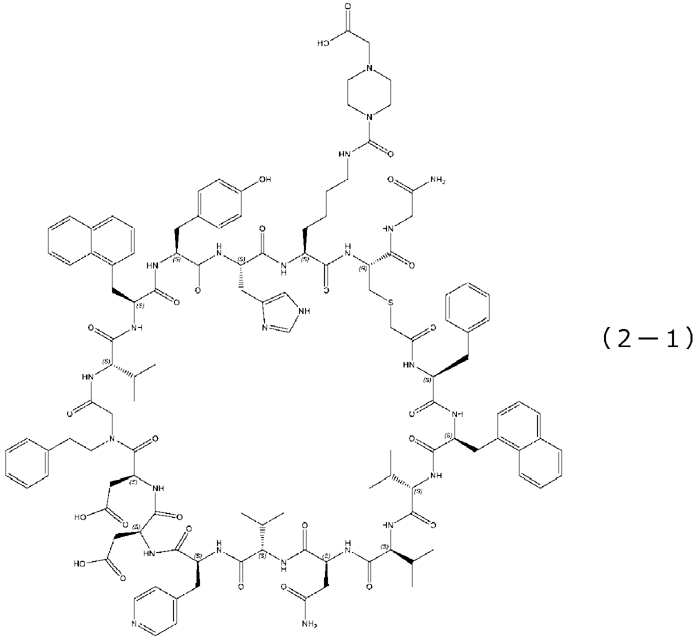

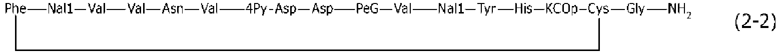

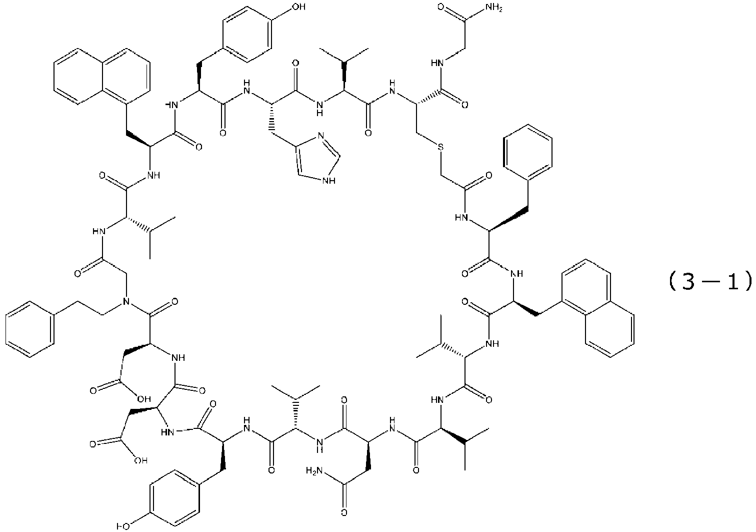

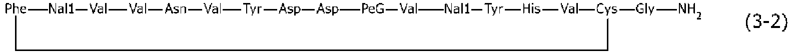

### [Experimental Example 2]

### (Culturing of human colon epithelial cells)

Human colon epithelial cells (including human colon stem cells) were dispersed in Matrigel (registered trademark, manufactured by BD Biosciences). Subsequently, the dispersion product was seeded into each well of a 48-well tissue culture plate and incubated at 37°C for 10 minutes to polymerize Matrigel (registered trademark).

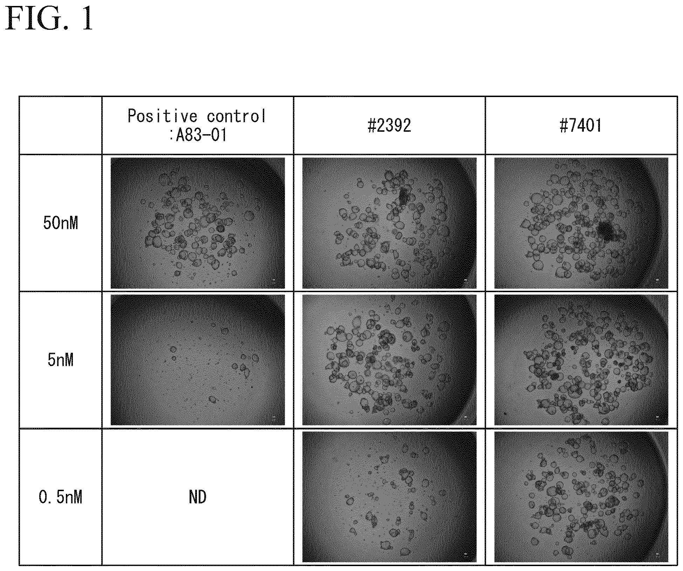

Subsequently, the culture medium (300 µL/well) prepared in Experimental Example 1 was overlaid on each well, and culturing was carried out at 37°C for 7 days in the presence of 5% by volume of CO₂. The culture medium was exchanged every two or three days. Subsequently, the inside of the wells after culturing for 7 days was observed with an optical microscope. A bright field image is shown in FIG. 1. In FIG. 1, "ND" indicates that data was not acquired.

As a result, it was clarified that organoids can be formed with an efficiency equal to or higher than that in a case where A83-01 was added by culturing human colon stem cells using a culture medium to which #2392 peptide and #7401 peptide were added.

### [Experimental Example 3]

### (Culturing of human hepatocytes)

Cryopreserved primary human hepatocytes in suspension (HEP187-S, manufactured by Biopredic International) were thawed in a water bath at 37°C, suspended in a 50 mL tube to which a serum-free culture medium (a culture medium obtained by adding HEPES, GLUTAMAX, and PENICILIN/STEREPTOMYCIN to Advanced DMEM/F 12) had been added, and centrifuged. After the centrifugation, the supernatant was removed, and then the resultant product was suspended in a serum-free culture medium to prepare a human hepatocyte suspension. 20,000 human hepatocytes were collected from the suspension, mixed with 50 µL of Matrigel (registered trademark, manufactured by BD Biosciences), seeded in a 24-well tissue culture plate, and incubated at 37°C for 10 minutes until Matrigel (registered trademark) was completely polymerized. After the polymerization, a culture medium (500 µL/well) prepared by adding each component to a basal medium to have the concentration shown in Table 4 below was overlaid, and culturing was carried out at 37°C in the presence of 5% by volume of CO₂. Advanced DMEM/F12 was used as a basal medium. The culture medium was exchanged every two or three days.

**[Table 4]**

| Component | Concentration |
|---|---|
| R-spondin1 (R-spondin1 CM) | 10 v/v% (to basal medium) |
| Wnt3a (Wnt3a CM) | 20 v/v% (to basal medium) |
| Noggin (Noggin CM) | 2 v/v% (to basal medium) |
| EGF | 50 ng/mL |
| FGF10 | 100 ng/mL |
| HGF | 25 ng/mL |
| Y-27632 | 10 µM |
| Forskolin | 10 nM |
| IL-6 | 100 ng/mL |
| A83-01 or #7401 peptide | 50 nM, 500 nM, or 5 µM |

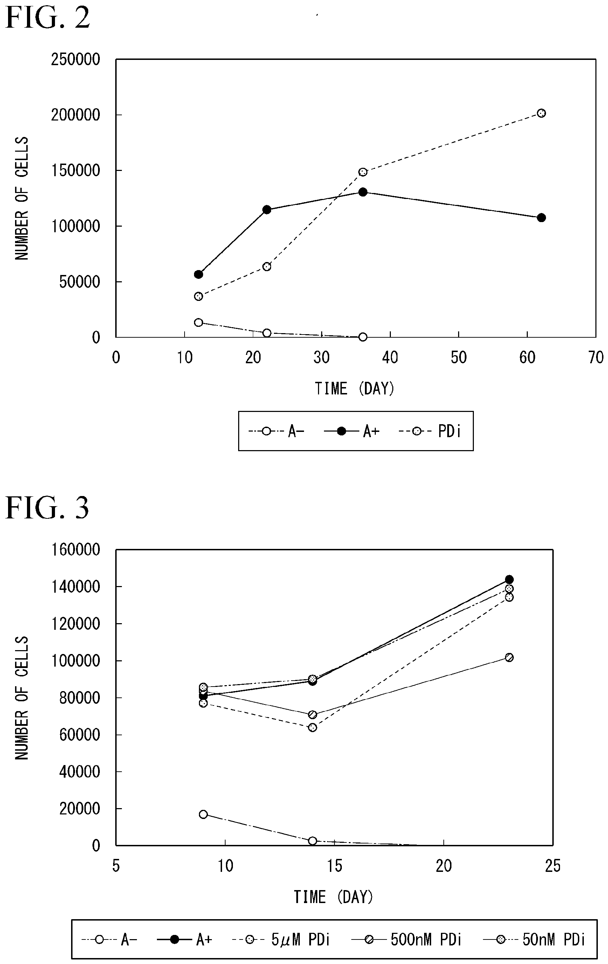

FIG. 2 is a graph showing a result of measuring the proliferation of cells. In FIG. 2, "PDi" shows a result in a case where 5 µM of the #7401 peptide was added to the culture medium, "A+" shows a result in a case where 5 µM of A83-01 was added to the culture medium, and "A-" shows a result in a case where neither #7401 peptide nor A83-01 was added to the culture medium.

From the result of FIG. 2, it is recognized that in a case where 5 µM of A83-01 is added to the culture medium, proliferation of cells is not observed after 35 days. From this result, it was suggested that it is likely that A83-01 is affected by metabolism by hepatocytes.

FIG. 3 is a graph showing a result of measuring proliferation of cells in a case where the concentration of the #7401 peptide was set to be low. In FIG. 3, "5 µM PDi" shows a result in a case where 5 µM of the #7401 peptide was added to the culture medium, "500 nM PDi" shows a result in a case where 500 nM of the #7401 peptide was added to the culture medium, "50 nM PDi" shows a result in a case where 50 nM of the #7401 peptide was added to the culture medium, "A+" shows a result in a case where 5 µM of the A83-01 was added to the culture medium, and "A-" shows a result in a case where neither the #7401 peptide nor the A83-01 was added to the culture medium.

From the result of FIG. 3, it was clarified that cells can sufficiently proliferate even in a case where the concentration of the #7401 peptide in the culture medium is 50 nM.

### [Experimental Example 4]

### (Evaluation of specificity of #7401 peptide by SMAD binding element (SBE) reporter assay)

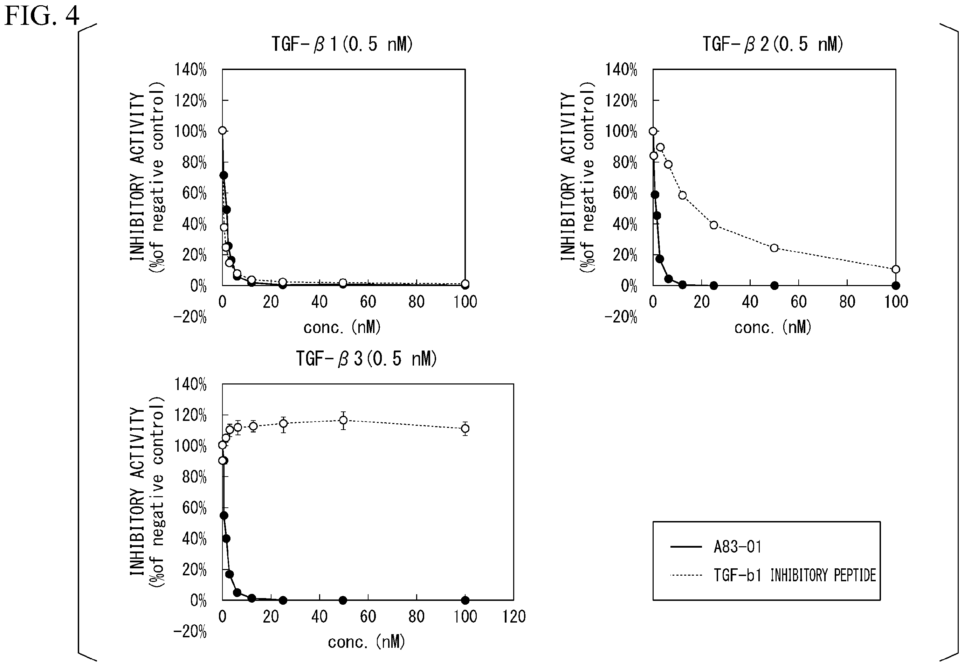

Reporter cells (TGF/SMAD Signaling Pathway SBE Reporter, Luciferase, HEK293 Recombinant Cell Line, manufactured by BPS Bioscience Inc.) were stimulated with 0.5 nM of various TGF-βs (TGF-β1 to TGF-β3), and A83-01 or #7401 peptide at the concentrations shown in FIG. 4, and after 20 hours, a signal was detected using the ONE-Glo^{™} Luciferase Assay System. In the present assay system, in a case where a TGF-β signal is transmitted into a nucleus, the luciferase which is a reporter gene is expressed, and a signal is detected. The result is shown in FIG. 4.

### [Experimental Example 5]

### (Examination of EGFR signal and p38 MAPK signal)

Upregulation of EGFR signal is known to induce cell proliferation. Therefore, the cell proliferative properties in a state where there was no EGFR signal were examined. In addition, it has been reported that A83-01 inhibits p38 MAPK signaling pathway (Vogt J., et al., The specificities of small molecule inhibitors of the TGF-beta and BMP pathways, Cell Signal., 23 (11), 1831-1842, 2011), and in addition, downregulation of p38 MAPK signal is known to be effective for long-term culture of the intestine (Sato T., et al., Long-term expansion of epithelial organoids from human colon, adenoma, adenocarcinoma, and Barrett's epithelium, Gastroenterology, 141 (5), 1762-1772, 2011). Therefore, culturing was examined in the presence or absence of the p38 MAPK signal.

To the basal medium (Advanced DMEM/F12), each component was added to the concentration shown in Table 5 below to prepare a culture medium. Human small intestinal epithelial cells (including human small intestinal stem cells) were dispersed in Matrigel (registered trademark, manufactured by BD Biosciences). Subsequently, the dispersion product was seeded into each well of a 48-well tissue culture plate and incubated at 37°C for 10 minutes to polymerize Matrigel (registered trademark).

**[Table 5]**

| Component | Concentration |
|---|---|
| HEPES | 10 nM |
| GlutaMAX | 2nM |
| Gibco^{™} Penicillin-Streptomycin (5,000 U/mL) | Penicillin 100 U/mL |
| (Thermo Fisher Scientific, Inc.) | Streptomycin 100 mg/mL |
| [Leucine15]-gastrin I | 10 nM |
| N-acetyl-L-cysteine | 1 mM |
| IGF1 | 100 ng/mL |
| FGF2 | 50 ng/mL |
| Wnt3a (Wnt3a CM) | 20 v/v% (to basal medium) |
| R-spondin1 (R-spondin1 CM) | 4 v/v% (to basal medium) |
| Noggin (Noggin CM) | 2 v/v% (to basal medium) |
| Gibco^{™} B27 supplement (Thermo Fisher Scientific, Inc.) | Appropriate amount |
| EGF | 0 ng/mL or 50 ng/mL |
| SB202190 | 0 µM or 10 µM |
| A83-01 or #7401 peptide | 50 nM, 500 nM, or 5,000 nM |

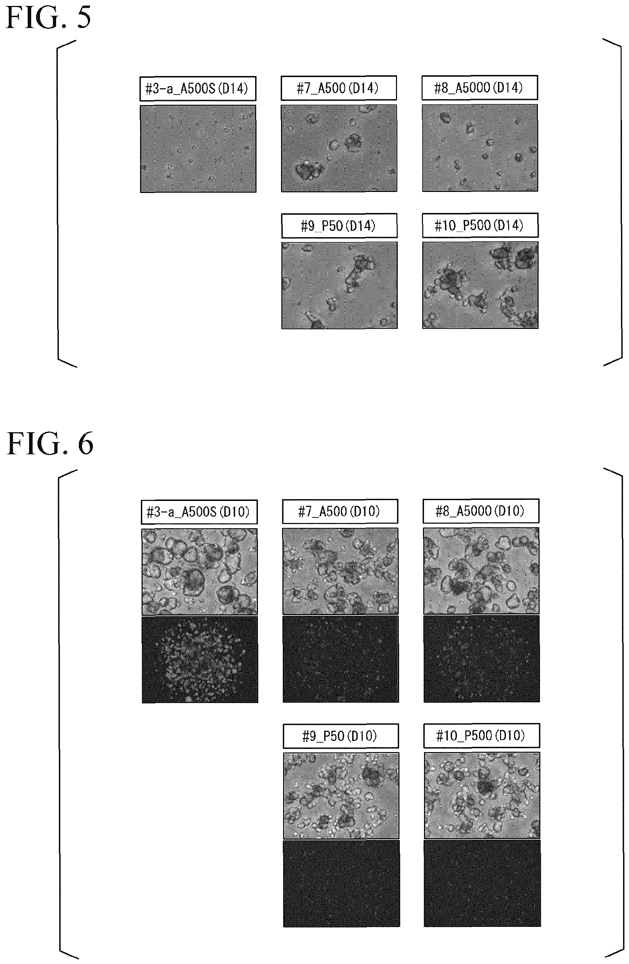

Subsequently, 300 µL/well of a culture medium was overlaid, and culturing was carried out at 37°C for 10 days in the presence of 5% by volume of CO₂. The culture medium was exchanged every two or three days. Cells were cultured for 10 days in a culture medium containing EGF and cultured for 14 days in a culture medium containing no EGF. Thereafter, bright field observation with an optical microscope in the well and Lgr5-tdTomato reporter assay were carried out. FIG. 5 shows a culture result in a culture medium containing EGF (50 ng/mL), and FIG. 6 shows a culture result in a culture medium containing no EGF. Among FIGS. 5 and 6, "#3-a_A500S" shows a culture result in a culture medium containing 500 nM of A83-01 and 10 µM of SB202190, "#7_A500" shows a culture result in a culture medium containing 500 nM of A83-01 and not containing SB202190, "#8_A5000" shows a culture result in a culture medium containing 5000 nM of A83-01 and not containing SB202190, "#9_P50" shows a culture result in a culture medium containing 50 nM of #7401 peptide and not containing SB202190, and "#10_P500" shows a culture result in a culture medium containing 500 nM of #7401 peptide and not containing SB202190.

"#3-a_A500S" of FIG. 5 shows a result showing inhibition of cell proliferative properties and colony forming properties by downregulation of p38 MAPK signal. In addition, the results of "#7_A500" and "#8_A5000" of FIG. 5 show that by using A83-01 the same as the result of "#3-a_A500S", the cell proliferative properties and the colony forming properties were concentration-dependently inhibited. Therefore, the result shows the possibility of p38 MAPK signaling pathway inhibition by A83-01.

The result of "#3-a_A500S" of FIG. 6 supports the result of Sato T., et al., Long-term expansion of epithelial organoids from human colon, adenoma, adenocarcinoma, and Barrett's epithelium, Gastroenterology, 141 (5), 1762-1772, 2011, which shows that the downregulation of p38 MAPK signal is effective for long-term culture of the intestine. "#7_A500" and "#8_A5000" of FIG. 6 have higher stem cell properties than "#9_P50" or "#10_P500", and in addition, "#8_A5000" has higher stem cell properties than "#7_A500", and thus there is shown a possibility of p38 MAPK signaling pathway inhibition by A83-01.

### [Experimental Example 6]

### (Formation of cerebral cortical organoid)

hiPSCs (PChiPS771 strain, Lot. A01QM28, manufactured by Reprocell Inc.) were subjected to feeder-free culture. Specifically, the hiPSCs were washed with phosphate buffered saline (PBS) and then dispersed into single cells using TrypLE Select (manufactured by Thermo Fisher Scientific, Inc.). Subsequently, the dispersed hiPSCs were seeded in a plastic culture dish coated with a human recombinant laminin fragment (product name: "iMatrix-511", manufactured by Nippi, Inc.) containing only an active site of laminin-511 and subjected to feeder-free culture in a StemFit AK02N culture medium (manufactured by Ajinomoto Co., Inc.) in the presence of Y27632 (a ROCK inhibitor, 10 µM). In a case where a 60 mm dish (for cell culture, manufactured by IWAKI & CO., LTD.) was used as the plastic culture dish, the number of seeded cells of the hiPSCs dispersed into single cells was 3 × 10⁴ cells/dish.

One day after seeding the cells, the culture medium was exchanged with a StemFit AK02N culture medium not containing Y27632. After that, the culture medium was exchanged with the StemFit AK02N culture medium not containing Y27632 once every 1 to 2 days. After that, 6 days after seeding the cells, the cells became 80% confluent.

Subsequently, an enlarged culture of the hiPSCs was subjected to a cell dispersion liquid treatment using TrypLE Select (product name, manufactured by Thermo Fisher Scientific, Inc.) and further dispersed into single cells by a pipetting operation. The enlarged culture of the dispersed hiPSCs was seeded to have a cell concentration of 1 × 10⁴ cells/well into a 96-well culture plate (product name "PrimeSurface 96V bottom plate", manufactured by Sumitomo Bakelite Co., Ltd.) in a culture medium (100 µL/well) prepared by adding each component to a basal medium ("DMEM/F-12, HEPES", product name, manufactured by Thermo Fisher Scientific, Inc.) to have the concentration shown in Table 6, and were subjected to float culture for 7 days in an incubator (37°C, 1 atmospheric pressure, CO₂ concentration 5 v/v%) to obtain aggregates of the hiPSCs.

**[Table 6]**

| Component | Concentration |
|---|---|
| KnockOut^{™} Serum Replacement | 25 v/v% (to basal medium) |
| (Thermo Fisher Scientific, Inc.) | |
| GlutaMAX (Thermo Fisher Scientific, Inc.) | Amount at which dilution concentration relative to basal medium is 200 times |
| MEM Non-Essential Amino Acids Solution (100×) (Thermo Fisher Scientific, Inc.) | Amount at which dilution concentration relative to basal medium is 100 times |
| 2-mercaptoethanol | Amount at which dilution concentration relative to basal medium is 100 times |
| Penicillin-streptomycin mixture solution (Nacalai Tesque, Inc.) | Amount at which dilution concentration relative to basal medium is 200 times |
| IWP-2 | 2 µM |
| SB-431542 or #7401 peptide | 1.25 µM, 2.5 µM, 5 µM, or 10 µM |

Subsequently, 230 µL/well of culture medium was removed from the well. Subsequently, 150 µL/well of a culture medium prepared by adding each component to the basal medium ("DMEM/F-12, HEPES", product name, manufactured by Thermo Fisher Scientific, Inc.)) to have the concentration shown in Table 7 was added thereto, and was subjected to float culture while stirring for 7 days in an incubator (37°C, 1 atmospheric pressure, CO₂ concentration 5 v/v%).

**[Table 7]**

| Component | Concentration |
|---|---|
| MEM Non-Essential Amino Acids Solution (100×) (Thermo Fisher Scientific, Inc.) | Amount at which dilution concentration relative to basal medium is 200 times |
| GlutaMAX (Thermo Fisher Scientific, Inc.) | Amount at which dilution concentration relative to basal medium is 100 times |
| Penicillin-streptomycin mixture solution (Nacalai Tesque, Inc.) | Amount at which dilution concentration relative to basal medium is 200 times |
| KnockOut^{™} Serum Replacement (Thermo Fisher Scientific, Inc.) | 25 v/v% (to basal medium) |
| Recombinant Human Wnt-3a (R&D systems) | 4 ng/mL |
| CHIR99021 | 1 µM |
| SB-431542 | 1 µM |
| Matrigel (registered trademark) | 30 v/v% |

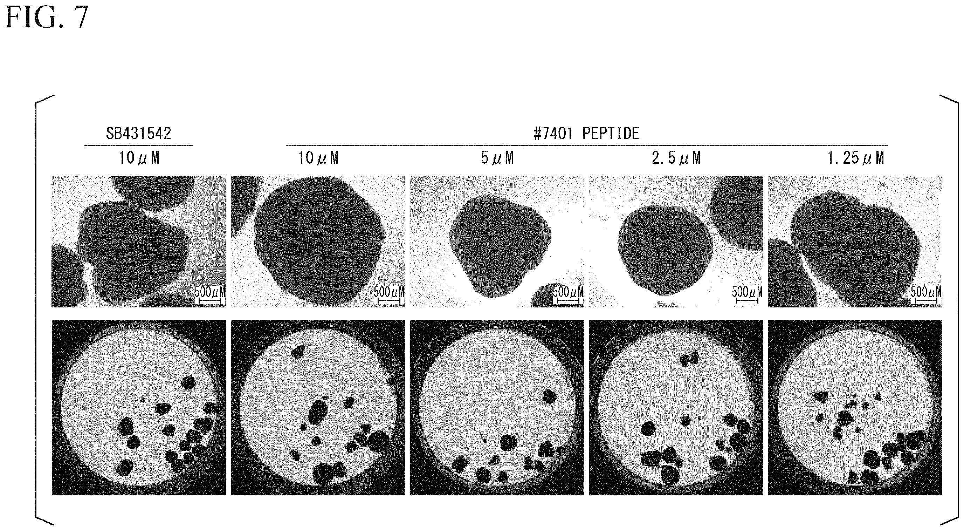

Subsequently, a content of the well was transferred to 50 mL Falcon (registered trademark) conical tube (manufactured by Corning Inc.) in which 10 mL of PBS was put. Subsequently, mixing with inversion was carried out 5 times, the supernatant was removed, and cell aggregates were recovered. 30 recovered cell aggregates and a culture medium (30 ml/well) prepared by adding each component to the basal medium ("DMEM/F-12, HEPES", product name, manufactured by Thermo Fisher Scientific, Inc.) to have the concentration shown in Table 8 were added to a single-use bioreactor (ABLE Corporation), and were subjected to float culture while stirring. The culture medium was exchanged every 4 days. 12 weeks after the start of float culture, cell aggregates (cerebral cortical organoid) in the single-use bioreactor were recovered and transferred to a 96-well culture plate. FIG. 7 is an optical microscope image showing a state of cell aggregates in a 96-well culture plate after float culture for 12 weeks. A lower part of FIG. 7 is an image obtained by imaging the entire well, and an upper part of FIG. 7 is an enlarged image. The scale bar is 500 µm.

**[Table 8]**

| Component | Concentration |
|---|---|
| MEM Non-Essential Amino Acids Solution (100×) | Amount at which dilution concentration relative to basal medium is 200 times |
| (Thermo Fisher Scientific, Inc.) | |
| 2-mercaptoethanol (Thermo Fisher Scientific, Inc.) | Amount at which dilution concentration relative to basal medium is 1,000 times |
| GlutaMAX (Thermo Fisher Scientific, Inc.) | Amount at which dilution concentration relative to basal medium is 100 times |
| Chemically Defind Lipid Concentrate (Thermo Fisher Scientific, Inc.) | Amount at which dilution concentration relative to basal medium is 100 times |
| N2 Supplement (100×) (Thermo Fisher Scientific, Inc.) | Amount at which dilution concentration relative to basal medium is 200 times |
| B27 serum-free supplement (Thermo Fisher Scientific, Inc.) | Amount at which dilution concentration relative to basal medium is 100 times |
| Penicillin-streptomycin mixture solution (Nacalai Tesque, Inc.) | Amount at which dilution concentration relative to basal medium is 200 times |

### [Industrial Applicability]

According to the present invention, it is possible to provide an organoid production method having excellent proliferative properties, a culture medium used for producing the organoid, an organoid obtained by the culturing, and a test substance evaluation method using the organoid.

## Claims

1. An organoid production method, comprising:
culturing a human stem cell in a culture medium containing a cyclic peptide having an amino acid sequence set forth in Formula (1) or a pharmaceutically acceptable salt of the cyclic peptide, 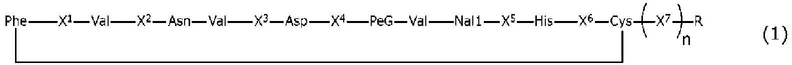 [in Formula (1),
X¹ represents Tyr, W6N, W7N, or Nal1,
X² represents Val, Lys, KCOp, or KCOm,
X³ represents Tyr or 4Py,
X⁴ represents Asp, Phe, KCOp, Glu, or KCOm,
X⁵ represents Tyr or 4Py,
X⁶ represents Val, Glu, KCOp, or KCOm,
X⁷ represents any amino acid residue,
R is absent or represents a C-terminal modification group,
n is an integer of 0 or 1,
PeG is N-(2-phenylethyl)-glycine,
Nal1 is β-(1-naphthyl)-L-alanine,
W6N is (S)-2-amino-3-(1H-pyrrolo[2,3-c]pyridin-3-yl)propanoic acid,
W7N is (S)-2-amino-3-(1H-pyrrolo[2,3-b]pyridin-3-yl)propanoic acid,
KCOp is N6-(4-(carboxymethyl)piperazine-1-carbonyl)-L-lysine,
KCOm is
N6-(methyl((2S,3R,4R,5R)-2,3,4,5,6-)pentahydroxyhexyl)carbamoyl)-L-lysine, and 4Py is 4-pyridyl-L-alanine].

2. The organoid production method according to Claim 1,
wherein a combination of X¹ to X⁷, R, and n in Formula (1) is a combination shown in Table 1 below.
**[Table 1]**
| Peptide number | X¹ | X² | X³ | X⁴ | X⁵ | X⁶ | X⁷ | R | n |
|---|---|---|---|---|---|---|---|---|---|
| 1 | Nal1 | Val | Tyr | Asp | Tyr | Val | Gly | NH₂ | 1 |
| 2 | Nal1 | Val | Tyr | Asp | Tyr | Val | - | NH₂ | 0 |
| 3 | Tyr | Val | Tyr | Asp | Tyr | Val | Gly | NH₂ | 1 |
| 4 | W6N | Val | Tyr | Asp | Tyr | Val | Gly | NH₂ | 1 |
| 5 | W7N | Val | Tyr | Asp | Tyr | Val | Gly | NH₂ | 1 |
| 6 | Nal1 | Val | 4Py | Asp | Tyr | KCOp | Gly | NH₂ | 1 |
| 7 | Nal1 | Val | 4Py | Asp | Tyr | Val | Gly | NH₂ | 1 |
| 8 | Nal1 | Lys | 4Py | Asp | Tyr | Val | Gly | NH₂ | 1 |
| 9 | W7N | Val | 4Py | Asp | Tyr | Val | Gly | NH₂ | 1 |
| 10 | Nal1 | Val | 4Py | Asp | 4Py | Val | Gly | NH₂ | 1 |
| 11 | Nal1 | KCOp | 4Py | Asp | Tyr | Val | Gly | NH₂ | 1 |
| 12 | Nal1 | Val | 4Py | KCOp | Tyr | Val | Gly | NH₂ | 1 |
| 13 | Nal1 | Val | 4Py | Asp | Tyr | KCOp | - | NH₂ | 0 |
| 14 | Nal1 | KCOm | 4Py | Asp | Tyr | Val | Gly | NH₂ | 1 |
| 15 | Nal1 | Val | 4Py | KCOm | Tyr | Val | Gly | NH₂ | 1 |
| 16 | Nal1 | Val | 4Py | Asp | Tyr | KCOm | Gly | NH₂ | 1 |
| 17 | W7N | Val | 4Py | KCOp | Tyr | Val | Gly | NH₂ | 1 |
| 18 | W7N | Val | 4Py | KCOm | Tyr | Val | Gly | NH₂ | 1 |
| 19 | Nal1 | KCOp | 4Py | Asp | Tyr | KCOp | Gly | NH₂ | 1 |
| 20 | Nal1 | KCOm | 4Py | Asp | Tyr | KCOp | Gly | NH₂ | 1 |
| 21 | Nal1 | Val | 4Py | Phe | Tyr | KCOp | Gly | NH₂ | 1 |
| 22 | Nal1 | Val | 4Py | KCOp | Tyr | KCOp | Gly | NH₂ | 1 |
| 23 | Nal1 | Val | 4Py | KCOm | Tyr | KCOp | Gly | NH₂ | 1 |
| 24 | Nal1 | KCOm | 4Py | Phe | Tyr | KCOp | Gly | NH₂ | 1 |
| 25 | Nal1 | KCOm | 4Py | KCOp | Tyr | KCOp | Gly | NH₂ | 1 |
| 26 | Nal1 | KCOm | 4Py | KCOm | Tyr | KCOp | Gly | NH₂ | 1 |
| 27 | Nal1 | Val | 4Py | Asp | Tyr | Glu | Gly | NH₂ | 1 |
| 28 | Nal1 | KCOm | 4Py | Asp | Tyr | Glu | Gly | NH₂ | 1 |
| 29 | Nal1 | KCOm | 4Py | Glu | Tyr | Glu | Gly | NH₂ | 1 |
| 30 | Nal1 | KCOm | 4Py | KCOm | Tyr | Glu | Gly | NH₂ | 1 |
| 31 | Nal1 | Val | 4Py | Asp | Tyr | KCOp | Gly-Lys | PEG-NH₂ | 1 |
| | | | | | | | | | |
|---|---|---|---|---|---|---|---|---|---|
| In Table 1, "PEG" represents polyethylene glycol chain. | | | | | | | | | |

3. The organoid production method according to Claim 1 or 2,
wherein in the amino acid sequence set forth in Formula (1), X¹ is Nal1, X² is Val, X⁴ is Asp, and X⁵ is Tyr.

4. The organoid production method according to any one of Claims 1 to 3,
wherein a bond between Phe and Cys in the amino acid sequence represented by Formula (1) is a bond via a thioether bond.

5. The organoid production method according to any one of Claims 1 to 4,
wherein X⁷ of the amino acid sequence represented by Formula (1) includes Gly.

6. The organoid production method according to any one of Claims 1 to 5,
wherein a total concentration of the cyclic peptide and the pharmaceutically acceptable salt of the cyclic peptide contained in the culture medium is 0.1 nM to 10 µM.

7. The organoid production method according to any one of Claims 1 to 6,
wherein the culture medium further contains a Wnt signal activator.

8. The organoid production method according to any one of Claims 1 to 7,
wherein the culture medium further contains an epidermal growth factor (EGF), a fibroblast growth factor (FGF), an insulin-like growth factor (IGF), a hepatocyte growth factor (HGF), or a combination thereof.

9. The organoid production method according to any one of Claims 1 to 8,
wherein the culture medium further contains a bone morphogenetic protein (BMP) inhibitor.

10. The organoid production method according to any one of Claims 1 to 9,
wherein the culture medium further contains a Rho-kinase (ROCK) signaling pathway inhibitor.

11. The organoid production method according to any one of Claims 1 to 10,
wherein the culture medium does not substantially contain an antagonist of ALK5, ALK4, ALK7, or a combination thereof.

12. The organoid production method according to any one of Claims 1 to 11,
wherein the human stem cell is cultured while being brought into contact with an extracellular matrix.

13. A culture medium for organoid production, comprising:
a cyclic peptide having an amino acid sequence set forth in Formula (1) or a pharmaceutically acceptable salt of the cyclic peptide, 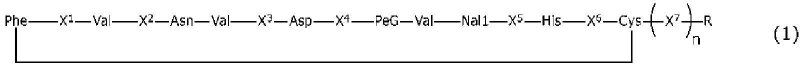 [in Formula (1),
X¹ represents Tyr, W6N, W7N, or Nal1,
X² represents Val, Lys, KCOp, or KCOm,
X³ represents Tyr or 4Py,
X⁴ represents Asp, Phe, KCOp, Glu, or KCOm,
X⁵ represents Tyr or 4Py,
X⁶ represents Val, Glu, KCOp, or KCOm,
X⁷ represents any amino acid residue,
R is absent or represents a C-terminal modification group,
n is an integer of 0 or 1,
PeG is N-(2-phenylethyl)-glycine,
Nal1 is β-(1-naphthyl)-L-alanine,
W6N is (S)-2-amino-3-(1H-pyrrolo[2,3-c]pyridin-3-yl)propanoic acid,
W7N is (S)-2-amino-3-(1H-pyrrolo[2,3-b]pyridin-3-yl)propanoic acid,
KCOp is N6-(4-(carboxymethyl)piperazine-1-carbonyl)-L-lysine,
KCOm is
N6-(methyl((2S,3R,4R,5R)-2,3,4,5,6-)pentahydroxyhexyl)carbamoyl)-L-lysine, and 4Py is 4-pyridyl-L-alanine].

14. An organoid obtained by the organoid production method according to any one of Claims 1 to 12.

15. A test substance evaluation method, comprising:
a step of bringing the organoid according to Claim 14 into contact with a test substance; and
a step of evaluating an influence exerted by the test substance on the organoid.
